# EUROPÄISCHE PATENTANMELDUNG

(11) **EP 0 693 484 A1**
(43) Veröffentlichungstag der Anmeldung: **24.01.1996**
(21) Anmeldenummer: 95110910.7
(22) Anmeldetag: 12.07.1995
(51) Int. Cl.: C07D 251/32, C07D 251/56

(54) **Verfahren zur Herstellung von Cyanursäure durch Abscheidung aus einem Isocyansäure-Ammoniak Gasgemisch**

(30) Priorität: 22.07.1994 AT 1454/94
(71) Anmelder: Chemie Linz GmbH, A-4021 Linz (AT)
(72) Erfinder: Schulz, Erich, A-4052 Ansfelden (AT); Häubl, Georg, Dr., A-4040 Linz (AT); Müllner, Martin, Dr., A-4020 Linz (AT)
(74) Vertreter: Kunz, Ekkehard, Dr.

(57) **Zusammenfassung**

Verfahren zur Herstellung von Cyanursäure durch Abkühlen eines Isocyansäure-Ammoniak-Gasgemisches mit einer Temperatur von 300 bis 480°C auf 200 bis 300°C, worauf Cyanursäure bei dieser Temperatur in einer zur Feststoffabtrennung aus Gasen geeigneten Vorrichtung abgeschieden und isoliert wird und nicht umgesetztes Isocyansäure-Ammoniak-Gasgemisch recycliert oder weiterverarbeitet wird.

## Beschreibung

Aus der Literatur sind bereits mehrere Herstellungsmöglichkeiten für Cyanursäure bekannt. So kann Cyanursäure beispielsweise auf dem Feststoffweg hergestellt werden, etwa durch Erhitzen von Harnstoff, wobei der Harnstoff auf einem Band durch eine beheizte Kammer bei Temperaturen zwischen 150 und 300°C geführt wird. Dabei entstehen jedoch eine Reihe von Nebenprodukten, sodaß die so gewonnene Cyanursäure zur Erzielung von Verkaufsqualität einer sauren Hydrolyse unterzogen werden muß. Eine weitere Herstellungsmöglichkeit ist der Lösungsmittelweg, bei dem Harnstoff in einem hochsiedenden Lösungsmittel pyrolysiert wird. In diesem Fall muß anschließend das verwendete Lösungsmittel abgetrennt und wiederum eine saure Hydrolyse durchgeführt werden, um eine ausreichende Qualität der Cyanursäure zu gewährleisten.
Aus DE 11 08 697 ist weiters ein Verfahren zur Herstellung von Cyanursäure bekannt, bei welchem Harnstoff in einen auf etwa 350° bis 700°C erhitzten Gasstrom eingebracht wird, die entstandene Cyanursäure von dem heißen Gas abgetrennt und restlicher Harnstoff rückgewonnen wird. Der Nachteil bei diesem Verfahren, sowie bei allen anderen direkt von Harnstoff ausgehenden Verfahren ist jedoch, daß die Cyanursäure Harnstoffverunreinigungen enthält, die bei verschiedenen Weiterverarbeitungen, etwa zur Herstellung von temperaturstabilen Flammschutzpigmenten, stören.

Aufgabe der vorliegenden Erfindung war es demnach ein Verfahren zu finden, das Cyanursäure, bevorzugt harnstoffarme Cyanursäure, auf einfache Weise und in hoher Qualität liefert.
Unerwarteterweise konnte diese Aufgabe durch Abscheidung von Cyanursäure aus einem Isocyansäure-Ammoniak Gasgemisch gelöst werden.

Gegenstand der vorliegenden Erfindung ist daher ein Verfahren zur Herstellung von Cyanursäure, das dadurch gekennzeichnet ist, daß ein Isocyansäure-Ammoniak-Gasgemisch mit einer Temperatur von 300 bis 480°C auf 200 - 300°C abgekühlt wird, Cyanursäure bei dieser Temperatur durch Trimerisierung der Isocyansäure in einer zur Feststoffabtrennung aus Gasen geeigneten Vorrichtung abgeschieden und isoliert wird und nicht umgesetztes Isocyansäure-Ammoniak Gasgemisch recycliert bzw. weiterverarbeitet wird.

Nach dem erfindungsgemäßen Verfahren wird Cyanursäure aus einem Isocyansäure-Ammoniak Gasgemisch durch Trimerisierung der Isocyansäure abgeschieden. Das zur Durchführung des erfindungsgemäßen Verfahrens nötige gasförmige Gemisch aus Isocyansäure und Ammoniak sollte bevorzugt 1 bis 50 Vol% an Isocyansäure enthalten. Besonders bevorzugt wird ein Gemisch mit 3 bis 30 Vol% an Isocyansäure eingesetzt. Geeignete Isocyansäure-Ammoniak Gasgemische werden beispielsweise durch Harnstoffzersetzung, wie sie etwa bei der Melaminsynthese durchgeführt wird oder wie beispielsweise in EP-A-0 124 704 beschrieben, erhalten. Die Temperatur eines nach beispielsweise EP-A-0 124 704 gewonnenen Isocyansäure-Ammoniak Gasgemisches beträgt etwa 300 bis 480°C. Zur Abscheidung von Cyanursäure wird das Gasgemisch, gegebenenfalls zur Kühlung verdünnt mit einem Trägergas, wie etwa Ammoniak, Stickstoff, Argon, Helium oder Kohlendioxyd, in eine geeignete Apparatur eingeleitet und auf 200 bis 300°C abgekühlt. Bevorzugt wird die Temperatur auf 230 bis 280°C abgesenkt. Als geeignete Apparatur zur Cyanursäuregewinnung kommen dabei Abscheidevorrichtungen, die zur Feststoffabtrennung aus Gasen geeignet sind, in Frage.
Dies können beispielsweise Waschtürme, Kühler, Zyklone, Abscheider wie etwa in DE 12 27 422 beschrieben, mechanische Abscheidevorrichtungen, wie etwa Kühlschabkristallisator, oder Heißgasfilter sein.
Bevorzugt werden Heißgasfilter zur Cyanursäureabscheidung eingesetzt. Geeignete Heißgasfilter können dabei verschiedene Ausführungsformen aufweisen. Bevorzugt wird ein zyklonähnlicher, tangential angeströmter Heißgasfilter, der im Zentrum mit Filtereinsätzen, etwa mit Keramikfilterkerzen oder -filz, einem hochtemperaturbeständigen Kunststoffgewebe, beispielsweise einem Polyimidfilz oder Polysulfongewebe, gesinterten Metallfritten, Metallgewebe oder mit Glasfritten bzw. Glasgeweben, versehen ist, verwendet.

In einer bevorzugten Ausführungsform wird das Isocyansäure-Ammoniak-Gasgemisch zuerst in eine Gasmischkammer, beispielsweise in einen Zyklon oder in eine mechanische Mischvorrichtung eingebracht, mit einem Kühlgas, wie etwa Ammoniak, Stickstoff, Kohlendioxyd oder einem anderen unter den Reaktionsbedingungen inerten Gas, vermischt und das so erhaltene Mischgas in eine nachgeschaltete Abscheidevorrichtung, bevorzugt in einen Heißgasfilter, eingeleitet.

In einer besonders bevorzugten Ausführungsform bilden die Gasmischkammer und die Abscheidevorrichtung eine Einheit. Es wird dabei ein wie oben definiertes Kühlgas durch einen oder mehrere weitere Einlässe in die Abscheidevorrichtung, bevorzugt in einen Heißgasfilter, eingetragen, wobei die Temperatur des Kühlgases so gewählt wird, daß die Gesamttemperatur in der Abscheidevorrichtung zwischen 200 bis 300°C beträgt.

Durch ein gezieltes Einleiten des Kühlgases kann ein Verkrusten der Heißgasfilterwand bzw. der Mischkammerwand vermieden werden. Ein weiterer Vorteil der Kühlgaseinleitung ist, daß die Kühlung des Reaktionsgases nicht vollständig über die Behälterwand erfolgen muß, sodaß die Wandtemperatur etwas höher als die Mischgastemperatur gehalten werden kann, wodurch ebenfalls eine Verkrustung vermieden wird.
Gewünschtenfalls kann die Abscheidung auch ohne ein zusätzliches Kühlgas durchgeführt werden. Wird kein Kühlgas verwendet, so wird das Isocyansäure-Ammoniak-Gasgemisch vorzugsweise vor Einleitung in die Abscheidevorrichtung über einen Kühler gekühlt.

Der Abscheidegrad und die Reinheit der Cyanursäure ist dabei abhängig von der Temperatur, der Vermischung bzw. der Mischgeschwindigkeit zwischen Kühlgas und Isocyansäure-Ammoniak-Gasgemisch, der Konzentration an Isocyansäure und von der Verweilzeit in der Gasmisch- bzw. Abscheidevorrichtung, die im Bereich zwischen einer Millisekunde und einige Minuten, bevorzugt zwischen 0,01 bis 20 Sekunden liegt. Diese Parameter können je nach gewünschter Reinheit der Cyanursäure variieren. Die am Filtereinsatz abgeschiedene Cyanursäure wird isoliert, etwa durch Abklopfen oder Filterrückspülung mittels Gas, und gegebenenfalls gereinigt, etwa durch Umkristallisieren.
Das nichtumgesetzte Isocyansäure-Ammoniak Gasgemisch kann anschließend wieder verwendet werden, beispielsweise kann nicht umgesetztes Mischgas im Kreislauf geführt und ohne Aufarbeitung oder Reinigung wieder als Ausgangsgas zur Cyanursäuregewinnung eingesetzt werden. Nicht umgesetztes Gasgemisch kann aber auch in einen Harnstoffzersetzer, beispielsweise als Trägergas für Harnstoff oder als Wirbelgas, eingebracht werden. Eine weitere Einsatzmöglichkeit ist beispielsweise die Zuführung des nichtumgesetzten Gasgemisches ohne Aufarbeitungs- oder Reinigungsschritte, nach Aufheizung auf etwa 300 bis 480°C, in einen Melaminherstellungsprozess, sowie die Verwendung als Ausgangsprodukt für andere von Isocyansäure ausgehenden Herstellungsverfahren.

Ebenso kann die durch Umkristallisation der Cyanursäure erhaltene Mutterlauge aufgrund des niedrigen Harnstoffgehaltes mehrere Male im Kreislauf geführt werden.
Das erfindungsgemäße Verfahren kann sowohl kontinuierlich als auch diskontinuierlich durchgeführt werden.

In einer bevorzugten Ausführungsform des erfindungsgemäßen Verfahrens wird die Cyanursäuregewinnung mit einem von Harnstoffzersetzung ausgehenden Melaminherstellungsprozeß gekoppelt. Dabei wird Harnstoff, wie etwa in EP-A-0 124 704 beschrieben, in der Wirbelschicht zu einem Isocyansäure-Ammoniak Gasgemisch zersetzt und ein Teil des Gasgemisches oder das gesamte Gasgemisch nach Verlassen der Zersetzungsapparatur in eine der oben erwähnten Abscheidungsapparaturen, bevorzugt in einen Heißgasfilter, eingeleitet. Gleichzeitig wird durch einen oder mehrere zusätzliche Einlässe ein Kühlgas eingebracht. Cyanursäure wird unter den oben angeführten Bedingungen abgeschieden, das nicht umgesetzte Isocyansäure-Ammoniak-Gasgemisch, das auch das Kühlgas enthält, wird anschließend dem Melaminherstellungsprozeß wieder zugeführt. Dabei kann das Gasgemisch entweder in den Zersetzer eingeleitet werden, oder das Gasgemisch wird auf 300°C - 480°C erhitzt und in einen Melaminkontaktofen eingebracht. Durch diese gekoppelte Verfahrensweise wird die Wirtschaftlichkeit des erfindungsgemäßen Vefahrens deutlich erhöht.

Mit dem erfindungsgemäßen Verfahren kann Cyanursäure in hoher Reinheit, die mindestens 90 %, und teilweise über 95 % beträgt, auf einfache Weise hergestellt werden.

### Beispiel 1:

In einen Heißgasfilter mit 3 l Volumen, bestehend aus einem beheizbaren Doppelmantelgefäß, das nach unten konisch verjüngt ist, einem beheizbaren Deckel, in den ein Keramikfiltereinsatz mit einem Porendurchmesser von 15 µm (Fa. Schuhmacher) eingeschraubt ist, einer Austragsvorrichtung am unteren Ende und mit 2 leicht versetzten Gaseinlässen am oberen Ende, wurden 2,2 Nm³ Pyrolysegas (hergestellt durch thermische Zersetzung von Harnstoff analog EP-A-0 124 704) mit einer Temperatur von 380°C binnen 1,5 Stunden mit 3 Nm³ eines Stickstoffstroms mit einer Temperatur von 200°C tangential eingeleitet, wobei die Wandtemperatur des Heißgasfilters 270°C betrug.
Die Isocyansäurekonzentration im Heißgasfilter betrug 6,8 Vol%, der Rest war Ammoniak und Stickstoff. Die Temperatur im Heißgasfilter wurde auf 270°C gehalten. Es wurden 60 g Feststoff, der sich aus 92,4 % Cyanursäure, 0,6 % Ammelid, 1,3 % Ammelin, 6,3 % Melamin und 0,0029 % Harnstoff zusammensetzte, aus dem Filter isoliert.
30 g Feststoff wurden in Wasser umkristallisiert, wodurch Cyanursäure mit einer Reinheit von >99,8 und einem Harnstoffgehalt von < 10 µg/g erhalten wurde.

Analog Beispiel 1 wurden weitere Versuche mit veränderten Parametern durchgeführt.

Die Ergebnisse, sowie die Parameter sind in Tabelle 1 zusammengefaßt.

Die Temperatur des Pyrolysegases (PG) betrug dabei immer 380°C, die Temperatur des Kühlgasstromes (KS) betrug 200°C, es wurden Stickstoff (N₂), Ammoniak (NH₃) und Kohlendioxid (CO₂) als Kühlgas verwendet.

In den Beispielen 2, 3, 9, 10 und 11 wurden Keramikfilterkerzen (Fa. Schuhmacher, Type: Dia-Schumalith F-40, mit einem Innendurchmesser von 28 mm und einem Außendurchmesser von 60 mm und gewellter Oberfläche) in den übrigen Beispielen Polyimidfilter (Fa. Lenzing, Type: P-84 Nadelfilz, Filterabmessungen 250 x 28 mm, Filterfläche: 220 cm²) verwendet.

Es werden weiters folgende Abkürzungen verwendet:
Betriebszeit: BZ
Wandtemperatur: WT
Isocyansäurekonzentration: IK
Mischgastemperatur im Heißgasfilter: MT
abgeschiedene Feststoffmenge: FS
Gehalt an Cyanursäure nach Umkristallisation: G.n.U.
nicht bestimmt: n.b.
Gehalt der Cyanursäure vor Umkristallisation: G.v.U.
Harnstoffgehalt vor Umkristallisation: HS.v.U
Harnstoffgehalt nach Umkristallisation: HS.n.U

**Tabelle 1**

| Bsp | PG (Nm³) | KS/Art (Nm³/h) | BZ (min) | WT (°C) | IK Vol% | MT (°C) | FS (g) | G.v.U. (%) | HS.v.U. % | G.n.U. % | HS.n.U. (µg/g) |
|---|---|---|---|---|---|---|---|---|---|---|---|
| 2 | 2,45 | 2,0/N₂ | 92 | 230 | 7,3 | 242 | 93 | 93,9 | 0,280>99,9 | | <20 |
| 3 | 10,80 | - | 158 | 240 | 10,3 | 260-276 | 185 | 95,6 | 0,100>99,8 | | 15 |
| 4 | 2,35 | 2,0/N₂ | 88 | 230 | 5,0 | 240 | 55 | 93,8 | 0,200 | n.b. | n.b. |
| 5 | 2,77 | 2,4/NH₃ | 79 | 230 | 4,8 | 251 | 44 | 92,1 | 0,075 | n.b. | n.b. |
| 6 | 4,12 | 1,2/CO₂ | 79 | 270 | 7,0 | 251 | 45 | 92,1 | 0,120 | n.b. | n.b. |
| 7 | 26,60 | - | 605 | 250 | 10,5 | 270-290 | 513 | 91,0 | 0,155 | n.b. | n.b. |
| 8 | 37,60 | 2,4/NH₃ | 556 | 250 | 3,0 | 250-260 | 103 | 91,8 | 0,100 | n.b. | n.b. |

Weitere Versuche wurden analog Beispiel 1 durchgeführt, mit dem Unterschied, daß das Pyrolysegas vor Einleiten in den Heißgasfilter über einen Kühler, ohne Kühlgasverwendung, abgekühlt wurde.
Pyrolysegastemperatur nach Kühler: TnK
Einleitzeit in Heißgasfilter: EZ

| Bsp. | PG (Nm³) | TnK (°C) | EZ (min) | WT (°C) | IK Vol% | MT (°C) | F (g) | G.v.U. % | HS.v.U. % |
|---|---|---|---|---|---|---|---|---|---|
| 9 | 7,1 | 280 | 160 | 230 | 14,1 | 260-280 | 265 | 85,0 | 0,900 |
| 10 | 8,1 | 290 | 157 | 250 | 14,6 | 270-285 | 190 | 92,5 | 0,042 |
| 11 | 7,9 | 280 | 162 | 240 | 14,3 | 270-285 | 152* | 96,2 | 0,013 |

| | | | | | | | | | |
|---|---|---|---|---|---|---|---|---|---|
| * Es wurden 152 g kristalliner Feststoff, sowie 70 g harte Kruste mit 79,3 % Gehalt an Cyanursäure aus der Abscheidevorrichtung isoliert. | | | | | | | | | |

## Patentansprüche

1. Verfahren zur Herstellung von Cyanursäure, dadurch gekennzeichnet, daß ein Isocyansäure-Ammoniak-Gasgemisch mit einer Temperatur von 300 bis 480°C auf 200 - 300°C abgekühlt wird, Cyanursäure bei dieser Temperatur durch Trimerisierung der Isocyansäure in einer zur Feststoffabtennung aus Gasen geeigneten Vorrichtung abgeschieden und isoliert wird und nicht umgesetztes Isocyansäure-Ammoniak Gasgemisch recycliert bzw. weiterverarbeitet wird.

2. Verfahren nach Anspruch 1, dadurch gekennzeichnet, daß die Isocyansäurekonzentration im Isocyansäure-Ammoniak Gasgemisch 1 - 50 Vol % beträgt.

3. Verfahren nach Anspruch 1, dadurch gekennzeichnet, daß die Isocyansäurekonzentration im Isocyansäure-Ammoniak Gasgemisch 3 - 30 Vol % beträgt.

4. Verfahren nach Anspruch 1, dadurch gekennzeichnet, daß das Isocyansäure-Ammoniak Gasgemisch durch thermische Harnstoffzersetzung erhalten wird.

5. Verfahren nach Anspruch 1, dadurch gekennzeichnet, daß Cyanursäure bei 230 - 280°C abgeschieden wird.

6. Verfahren nach Anspruch 1, dadurch gekennzeichnet, daß Cyanursäure in einem Heißgasfilter abgeschieden wird.

7. Verfahren nach Anspruch 1, dadurch gekennzeichnet, daß Cyanursäure in einem zyklonähnlichen, tangential angeströmten Heißgasfilter, der im Zentrum mit Filtereinsätzen versehen ist, abgeschieden wird.

8. Verfahren nach Anspruch 1, dadurch gekennzeichnet, daß das Isocyansäure-Ammoniak-Gasgemisch in einer Gasmischkammer mit Kühlgas vermischt wird, das so erhaltene Mischgas in einen Heißgasfilter eingeleitet und Cyanursäure abgeschieden wird.

9. Verfahren nach Anspruch 1, dadurch gekennzeichnet, daß das Isocyansäure-Ammoniak-Gasgemisch im Heißgasfilter mit Kühlgas vermischt wird, das durch einen oder mehrere weitere Einlässe eingebracht wird.

10. Verfahren zur Herstellung von Cyanursäure gekoppelt mit einem von Harnstoffzersetzung ausgehenden Melaminherstellungsprozeß, dadurch gekennzeichnet, daß Harnstoff in einer Wirbelschicht bei 300 - 480°C zu einem Isocyansäure-Ammoniak Gasgemisch zersetzt wird, ein Teil des Gasgemisches oder das gesamte Gasgemisch nach Verlassen der Zersetzungsapparatur in eine Abscheidevorrichtung geleitet und gegebenenfalls unter gleichzeitigem Einbringen eines Kühlgases Cyanursäure bei einer Temperatur von 200 - 300°C abgeschieden wird, worauf das nicht umgesetzte Isocyansäure-Ammoniak Gasgemisch entweder in die Zersetzungsapparatur wieder eingebracht, oder auf eine Temperatur von 300 - 480°C aufgeheizt und in einem Melaminkontaktofen zu Melamin verarbeitet wird, oder als Ausgangsprodukt für andere von Isocyansäure ausgehenden Herstellungsverfahren verwendet wird.
